# EUROPEAN PATENT APPLICATION

(11) **EP 2 103 310 A1**
(43) Date of publication of application: **23.09.2009**
(21) Application number: 08153036.2
(22) Date of filing: 19.03.2008
(51) Int. Cl.: A61K 38/48

(54) **Method for the prevention or treatment of ischemia reperfusion injury.**

(71) Applicant: Universiteit Maastricht, 6211 LK Maastricht (NL)
(72) Inventor: Nicolaes, Gerardus Anna Franciscus, 6265 BE, SINT GEERTRUID (NL); Rosing, Jan, 6301 VK, VALKENBURG (NL); Cate, Ten, Hugo, 6212 XB, MAASTICHT (NL); Dahlbäck, Björn Ulfson, 217 74, MALMÖ (SE)
(74) Representative: Habets, Winand

(57) **Abstract**

The invention is in the field of thrombosis, haemostasis, cardiology, medicinal chemistry and/or pharmacology. More particularly, it relates to the use of a mutant activated protein C (APC) or protein C for preventing or treating ischemia reperfusion injury. A method is provided for treating or preventing reperfusion injury following an ischemic event that comprises administering to a subject a pharmaceutical preparation comprising an S360A APC and a pharmaceutically acceptable carrier.

## Description

### Field of the invention

The invention is in the field of thrombosis, haemostasis, cardiology, medicinal chemistry and/or pharmacology. More particularly, it relates to the use of a mutant activated protein C (APC) for the prevention or treatment of ischemia reperfusion injury. A method is provided for treating or preventing reperfusion injury following an ischemic event that comprises administering to a subject a pharmaceutical preparation comprising an S360A APC or a precursor thereof. Compositions for use in the present invention include S360A APC or a precursor thereof and an acceptable pharmaceutical carrier.

### Background of the invention

Ischemic heart disease and stroke remain the most common causes of death in the industrial world. In the United States, acute myocardial infarction (AMI) and stroke are annually responsible for about 530,000 (http://www.wrongdiagnosis.com/h/heart disease/deaths.htm) and 300,000 deaths, respectively. Both ischemic heart disease and stroke are a leading cause of hospital admissions and long-term disabilities. Accordingly, the socio-economic impact of these diseases and their burden on society is practically immeasurable.

AMI occurs upon thrombotic occlusion of one or more coronary arteries that are affected by atherosclerosis. When the occlusion persists beyond a certain period (usually minutes) myocardial ischemia may become critical and cardiomyocyte damage occurs. The latter is known as AMI, characterized by myocardial necrosis which is measurable by a rise in cardiac enzymes in blood, including troponin T and creatine phosphokinase. The functional consequence is acute heart dysfunction due to impaired pump activity, usually also influenced by rhythm abnormalities due to ischemia of the conduction pathways in the heart.

Ischemic strokes are typically caused by occlusions of the blood vessels to the brain or within the brain. With complete occlusion, arrest of cerebral circulation causes cessation of neuronal electrical activity within seconds. Within a few minutes after the deterioration of the energy state and ion homeostasis, depletion of high energy phosphates, membrane ion pump failure, efflux of cellular potassium, influx of sodium chloride and water, and membrane depolarization occur. Irreversible damage results if the occlusion persists for more than five to ten minutes.

The condition induced by cessation of blood flow and oxygen delivery to a tissue is known as ischemia. Substantial reductions of oxygen delivery induce a condition known as hypoxia. Both prolonged ischemia and hypoxia can result in the loss of function of the tissue and even cell death. There are numerous conditions, both natural and iatrogenic, that cause ischemia and hypoxia including, but not limited to, occlusive vascular disease, coronary thrombosis, cerebrovascular thrombosis, aneurysm rupture, general hemorrhage, crush injury, sepsis, severe cutaneous burns, vasculo-occlusive surgical techniques (such as spinal ischemia during thoracoabdominal aneurysm surgery), cardiopulmonary bypass procedures, organ transplantation, cardiopulmonary collapse (sudden cardiac death), and suffocation.

As flow drops below the ischemic threshold of 23 ml/100g/minute, symptoms of tissue hypoxia develop. Severe ischemia may be lethal. When the ischemia is moderate, it will result in "penumbra." In the neurological context, penumbra refers to a zone of brain tissue with moderate ischemia and paralyzed neuronal function, which is reversible with restoration of adequate perfusion. The penumbra forms a zone of collaterally perfused tissue surrounding a core of severe ischemia in which an infarct has developed. In other words, the penumbra is the tissue area that can be saved, and is essentially in a state between life and death.

Conventional treatment for ischemia and hypoxia is to restore blood flow and oxygen delivery to normal levels, either by increasing general oxygenation or by removing the cause of the vascular blockage. Restoration of blood flow results in improved outcomes when compared to situations wherein ischemia or hypoxia are maintained for longer periods of time. However, it is well recognized that the restoration of blood flow and oxygen delivery can cause additional cell death and loss of function independent of the damage caused by ischemia or hypoxia. This additional damage induced by the restoration of blood flow and oxygen delivery is known as ischemia reperfusion injury.

Portions of the injured tissue in the penumbra can be killed or further injured by the re-entry of oxygen or other substances into the area affected by the ischemia. In view of this phenomenon, the extent of tissue damage resulting from ischemia is determined both by the time required to achieve opening of an occluded vessel and by the series of reactions that follow as a result of reperfusion and the re-entry of oxygen to the affected tissue.

Ischemic reperfusion injury is especially problematic in organ transplantation because the harvested organ is removed from the donor body, isolated from a blood source, and thereby deprived of oxygen and nutrients for an extended period of time (U. S. Patent 5,912,019). In fact, one of the most critical problems in kidney transplantation today is the relatively high incidence of delayed graft function (DGF) due to acute tubular necrosis (ATN) after surgery.

For example, DGF affects 20-35% of kidney transplants in many transplant centers and is the most common complication of the immediate postoperative period in renal transplantation. Although the incidence and definition of DGF vary among transplant centers, the consequences are uniform: prolonged hospital stay, additional invasive procedures, and additional cost to the patient and health-care system. DGF impacts both the individual patient and the infrastructure for organ procurement and sharing because of the drain it places on the available organ supply. DFG also elevates the risk of early acute rejection episodes and increases early graft loss from chronic rejection (Koning et al., Transplantation 63: 1620-1628,1997, Ojo et al., Transplantation. 63: 968-974,1997, Matas et al., Transplantation, 69: 54-58, 2000).

The paradoxical tissue damage caused by ischemia reperfusion injury appears to be similar to an acute inflammatory condition, resulting from the adherence of inflammatory cells to the reperfused tissues, activation of these inflammatory cells and the subsequent generation of free radicals (Granger et al. Ann. Rev. Physio., 57, 311-332, 1995 ). The generation of free radicals and other cytotoxic biomolecules within reperfused tissue can induce cell death by either necrosis or by activation of the apoptosis pathway.

As a pathogenesis for development of the myocardial ischemia or ischemia reperfusion injury, an intracellular ATP deficiency theory, a calcium overloading theory and a free radical theory have previously been widely proposed.

Current treatments for ischemia reperfusion injury only adequately treat the ischemic damage by restoring blood flow and oxygenation. However, the damage caused by reperfusion injury is generally under-treated. Investigational treatments for ischemia reperfusion include the use of adenosine and adenosine analogs as well as inhibition of the sodium-calcium exchange pump on the ischemic myocytes. These therapies, however, are not sufficiently adequate. For example, the use of adenosine and adenosine analogs is burdened by the undesirable effects of depressor activity and bradycardia. Similarly, inhibition of the sodium-calcium exchange pump on the ischemic myocytes is inadequate because it does not prevent or treat the inflammatory conditions or the direct stimulation of apoptosis.

In other studies, N-acetyl cysteine (Koeppel et al., Eur. Surg. Res., 28 : 270-277, 1996), nitric oxide donors (Lopez-Neblina et al., Transplantation, 61: 179-183, 1996) and inhibitors of endothelin-1 converting enzyme (Vemulapalli et al., Pharmacology, 47: 188-193, 1993, Bird et al., Pharmacology, 50: 9-23, 1995) have been tested as therapeutic agents against ischemia reperfusion injury with limited success. Some protective effect has been shown for a combination therapy of N-acetyl cysteine, sodium nitroprusside and phosphoramidon against ischemia reperfusion injury in rat kidney after 90 min of normothennic ischemia (Dobashi et al., Mol. Cell Biochem., 240: 9-17, 2002) and also in dog kidney (Sekhon et al., Brain Res., 971: 1-8, 2003, Sekhon et al., J. Nephrol., 16: 63-74, 2003). 5-amino-4 imidazole carboxamide riboside (AICAR) has been shown to afford sustained protection against myocardial ischemia-reperfusion injury (A. M. Alkhulaifi and W. B. Pugsley, Br. Heart J. 73: 304-305, 1995, Galinanes et al., J. Thorac. Cardiovasc. Surg., 110: 752-761, 1995) where it is thought to increase regional blood flow by increasing local adenosine concentration. It can inhibit neutrophil activation (Mathew et al., J. Tizorac. Cardiovasc. Surg., 109: 448-456, 1995) and suppress platelet aggregation (Bullough et al., J. Clin. Invest., 94: 1524-1532, 1994).

Recently it has been proposed that patients at risk of damage to blood vessels or tissue in various organs caused by apoptosis might benefit from treatment with variants (mutants) of recombinant activated protein C (APC) or recombinant protein C which is a pro-drug, capable of being converted to APC (Kerschen et al., J. Exp. Med. 2439 - 2449, 2007). Such mutants were shown to have substantial reductions in their anticoagulant activity but retained normal levels of anti-apoptotic activity (Mosnier et al., Blood, 104; 1740-1744, 2004). Two examples of such recombinant APC mutants were provided (KKK191-193AAA-APC and RR229/230AA). Remarkably, in these studies S360A APC did not show any inhibition of apoptosis.

Despite of all the above solutions, there remains a need for new pharmaceutically acceptable compounds and compositions for preventing, limiting or treating ischemia reperfusion injury.

### Summary of the invention

It is therefore an object of the present invention to provide a method for preventing or treating reperfusion injury to tissue.

It is also an object of the present invention to provide a method for preventing or treating ischemia, infarction or brain edema.

It is also another object of the present invention to provide a method for improving microcirculation.

It is yet another object of the present invention to provide a method for preventing or treating a reperfusion injury that follows ischemic events. Such injury is also termed ischemia reperfusion injury herein.

It is yet another object of the present invention to provide a method for preventing or treating the reperfusion injury that follows ischemic events by administering a pharmaceutical composition.

It is still another object of the present invention to provide a method for preventing or treating ischemia and the attendant reperfusion injury resulting from events following ischemia by administering a pharmaceutical composition.

It is a further object of the present invention to prevent or treat ischemia and reperfusion injury resulting from surgical procedures, including transplants.

The above objects of the invention are met by providing an activated protein C (APC) with a reduced proteolytic activity.

In accordance with one aspect of the present invention, a method is provided for preventing or treating reperfusion injury that comprises administering to a subject a pharmaceutical preparation comprising an effective dose of activated protein C with a reduced proteolytic activity or a precursor thereof and a pharmaceutically acceptable carrier.

In other words, the invention provides a method for treating ischemia reperfusion injury in a mammal by administering a therapeutically effective amount of APC with reduced proteolytic activity or a precursor thereof to patients in need of such a treatment.

The invention also provides a method as described above wherein the APC with reduced proteolytic activity or a precursor thereof is a recombinant APC with reduced proteolytic activity or a precursor thereof.

The invention also provides a method as described above wherein the APC with reduced proteolytic activity or a precursor thereof is mutated in the active center of the serine protease domain of that APC or its precursor.

The invention also provides a method as described above wherein the APC with reduced proteolytic activity or a precursor thereof is S360A APC or S360A protein C.

As used herein, the phrase "activated protein C with a reduced proteolytic activity or a precursor thereof" is to be interpreted as activated protein C with a reduced proteolytic activity or its precursor protein C, which upon activation would generate activated protein C with a reduced proteolytic activity. Such molecules are known in the art and the skilled person will find ample guidance in the literature on how to obtain such molecules. Synthesis of these molecules by recombinant techniques is preferred from the perspective of safety and reproducibility.

In accordance with another aspect of the present invention, there is provided a method for the prevention or treatment of reperfusion injury resulting from ischemia and/or surgical procedures, including transplants. This treatment entails administering an effective dose of activated protein C with a reduced proteolytic activity or its precursor to patients who have or will undergo surgical procedures including transplantation.

In the case of transplantation, the administration of activated protein C with a reduced proteolytic activity or its precursor to tissue or organ recipients protects the tissue or organ to be transplanted, as well as the tissues and organs surrounding the surgical area of the recipient. Likewise, administration of activated protein C or protein C with a reduced proteolytic activity to the tissue or organ donor protects the tissue or organ to be transplanted as well as the tissues and organs surrounding the surgical area of the donor.

Other objects, features and advantages of the present invention will become apparent from the following description and figures.

### Detailed description of the invention

Human activated protein C (APC) is the major proteolytic regulator of thrombin formation in vivo. APC is known to act as a serine protease and exerts its proteolytic activity toward the protein cofactors FVa and FVIII. The cleavage of FVa and FVIIIa is a multistep proteolytic process that involves initial formation of an enzyme-substrate complex, followed by cleavage of the substrate and subsequent dissociation of the product from the enzyme. In the Examples section, we present in vivo experimental data that show that ischemia reperfusion injury in mice can be prevented and treated by administering a recombinant activated protein C with a reduced proteolytic activity.

The serine protease domain of activated protein C consists of 240 residues. The three most important residues (His-211, Asp-257, and Ser-360) define the functional role of a serine protease. It is common, in serine protease domains of both coagulant and anticoagulant serine proteases in the blood coagulation cascade, to observe the same global fold. The catalytic residues are located at the junction between two interconnected six-stranded beta-barrel domains. The serine protease domain of activated protein C, along with trypsin, coagulation factors II, VII, IX, and X, possess a calcium ion binding site (Perera et al., Biophys. J., 79, 2925-2943, 2000)

In the present study we exemplify the effects of an activated protein C with a reduced proteolytic activity in that we used a recombinant APC in which the active proteolytic site has been mutated. In more detail: we used a recombinant APC comprising an Alanine residue at the active site position 360 where a Serine residue occurs in the wild-type APC (S360A APC), which results in loss of the proteolytic activities of APC.

Various other mutants APCs may be used in the method according to the invention, as long as these mutants have reduced proteolytic activity. The term "reduced proteolytic activity" is used herein to indicate that the mutant APC should have a proteolytic activity that is less than the activity of a wild type APC. Such may be a 10% reduction in the proteolytic activity, but it is preferred to reduce the activity with 20% or more, such as 40%, 60%, 80%, 90%, 95% or even more. It is especially preferred to use a mutant with a substantially no proteolytic activity. The term "substantially no proteolytic activity" is used herein to indicate that the proteolytic activity of a certain mutant APC is not detectable within the margins of error using a conventional assay for the proteolytic activity of activated protein C. Such assays are known in the art.

Preferred examples of such APC molecules with reduced proteolytic activity may be APC molecules with a mutation at one of the residues defining the active site, i.e. His-211, Asp-257, and Ser-360. It will be understood that also mutations at other positions could lead to APC molecules with reduced proteolytic activity

It may also be envisaged that instead of an APC with reduced proteolytic activity, a precursor of APC is used that leads to an APC with reduced proteolytic activity, after activation of the precursor. Such a precursor could be protein C which after activation yields an APC with a reduced proteolytic activity, for instance caused by a mutation in the active site of the serine protease domain, such as a mutation at one of the residues defining the active site, i.e. His-211, Asp-257, and Ser-360, such as S360A.

Mutant APC with reduced proteolytic activity or a precursor thereof may be produced by various techniques known in the art. For example, one or more of the three most important residues (His-211, Asp-257, and Ser-360) may be mutated by using recombinant DNA techniques. A particularly useful mutant is S360A wherein a Serine residue at position 360 has been replaced by an Alanine residue. Mutant S360A APC does not exhibit a detectable proteolytic activity in the assays known in the art.

We have shown here in *in vivo* experiments that S360A APC showed greater potency in infarct size reduction than no treatment or treatment with comparable amounts of wild-type APC.

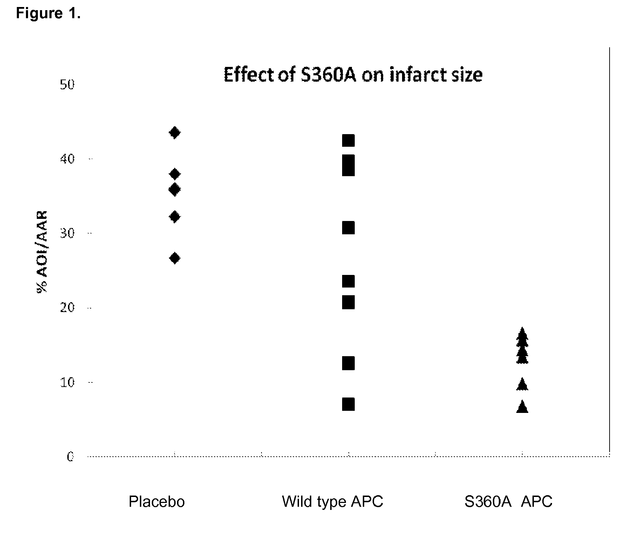

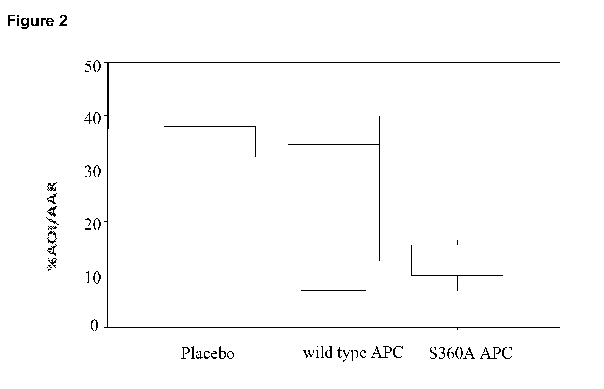

When injected in mice during an occlusion-reperfusion experiment, the S360A APC molecule appears to have increased efficacy, as compared to a placebo or to a similar amount of wild-type APC, as is evidenced by the reduction in infarct size (fig. 1). As is evident from the figure, the effect of the administration of the mutant APC is prominent and significant (figure 2) when measured after 2 hours of reperfusion.

Hence, the invention relates to a method for preventing, limiting, or treating ischemia reperfusion injury in a mammal, comprising the steps of identifying a mammal that has undergone an ischemic event, or in which an ischemic event is imminent or is at risk for having an ischemic event and administering a therapeutically effective or prophylactically effective amount of APC with reduced proteolytic activity.

Advantageously, in this method, a recombinant APC is used with reduced proteolytic activity. An APC with reduced proteolytic activity may advantageously be obtained by mutating the active center of the serine protease site of that APC. One preferred example of such a mutant is S360A APC.

The invention also relates to the use of an APC with reduced proteolytic activity or its precursor for the preparation of a pharmaceutical composition for the prevention or treatment of ischemia reperfusion injury. Advantageously, this use involves a recombinant APC with reduced proteolytic activity or its precursor. An APC with reduced proteolytic activity or its precursor may advantageously be obtained by mutating the active center of the serine protease site of that APC or its precursor. One preferred example of such a mutant is S360A APC.

In a different wording, the invention relates to an activated protein C with a reduced proteolytic activity or its precursor for use in treating or preventing ischemia reperfusion injury. Advantageously, the activated protein C with a reduced proteolytic activity or its precursor is a recombinant APC with reduced proteolytic activity or its precursor. An APC with reduced proteolytic activity or its precursor may advantageously be obtained by mutating the active center of the serine protease site of that APC or its precursor. One preferred example of such a mutant is S360A APC.

The invention also relates to the use of an APC with reduced proteolytic activity or its precursor for the preparation of a pharmaceutical composition for the prevention of ischemia reperfusion injury by administering said pharmaceutical composition to a subject at risk for ischemia reperfusion injury.

Without wanting to be bound by theory, the following is provided as a possible explanation for a mechanism of the effects observed in this study.

Plasma-circulating APC is known to be inhibited by circulating serine protease inhibitors (Serpins) such as protein C inhibitor (PCI) and α1-antitrypsin that are proteolysed by APC during inhibition. However, since Ser360Ala lacks proteolytic activity, this APC variant is not inhibited by serpins and hence has a prolonged half-life in plasma.

APC, a serine protease, is able to proteolyse its substrates via the so-called acylenzyme mechanism (Enzyme structure and mechanism, Alan Fersht, isbn 0716716143). This mechanism includes a number of discrete and ordered steps:
1. association of enzyme and substrate, resulting in the formation of a non-covalent enzyme-substrate complex that is held together by physical forces of attraction
2. Attack of the active site serine residue hydroxyl group on the substrate to give the tetrahedral reaction intermediate, a serine ester intermediate.
3. Collapse of the tetrahedral intermediate to form the acylenzyme
4. Hydrolysis of the acylenzyme which forms the enzyme-product complex
5. Dissociation of the product, resulting in free product and enzyme

Besides the active site Ser, also the active site His57, Asp102 and Gly193 (Chymotrypsinogen numbering) residues are involved in the formation and stabilization of reaction intermediates during enzyme catalysis. In case of human APC these residues are: Ser360, His211, Asp257 and Gly358. The binding site for a polypeptide substrate like FVa or FVIIIa is formed by a series of sub-sites on the surface of the enzyme, that may be outside the active site (see also Nicolaes et al, Eur J Biochem. 2004 Jul;271(13):2724-36.). Therefore, for the formation of a non-covalent enzyme-substrate complex, the presence of a functional catalytic site in the enzyme is not a prerequisite. However, in the absence of any of the active site residues, proteolysis of the substrate will not occur and the enzyme may remain bound (reversibly) to the substrate via non-covalent forces in the enzyme-substrate complex, thereby inhibiting the substrate.

Inhibition by active site mutated APC is reversible. This implies that at sufficiently high levels of physiogical ligands of FVIIIa/FVa, such as for instance FIXa and FXa respectively, natural ligands are able to compete with the mutant APC, which results in a regain of FVIIIa/FVa cofactor function (and normal coagulation).

### Legend to the figures

Figure 1: Influence of APC with reduced proteolytic activity on infarct size.
   All mice underwent ischemia for 1 hour followed by 2 hours of ischemia. After inducing the infarct, wild type APC, recombinant S360A APC or a placebo were administered. Abbreviations used: wtAPC = wild type APC; APC mut = S360A APC; AOI = area of infarction, the total afflicted area of the hart attributable to the infarct; AAR = area at risk, the total area of the hart that is provided with blood by the infarcted vessel.
Figure 2: Average and standard deviations of the data shown in figure 1.
   Shown is the effect of APC on the observed AOI/AAR ratio in the Mouse Myocardial Ischemia Reperfusion Model as described herein. AOI/AAR were measured in mice receiving placebo (Placebo), wild type APC or the S360A APC variant. The lines in the boxes indicate the median. The lower and upper edges correspond to 25th and 75th percentiles, respectively. The whiskers show the minimum and maximum values that do not extend more than 1.5-times the interquartile range.

### Examples

### Example 1: Mouse Myocardial Ischemia Reperfusion Model

All animal experiments were approved by the Animal Ethics Committee of the Maastricht University. Male C57Black/6 mice (Charles River Laboratories, Sulzfeld, Germany), 8 weeks old and 22 to 28 g, were housed under normal conditions: temperature was kept constant at 20-24 °C and food and water were provided ad libitum. Myocardial I/R was induced according to the method of Jong et al. (Comp Med,. 53(5); 522-526, 2003). Briefly, mice were anesthetized with isoflurane (induced with 3-4 % and maintained with 1.5-2.5 %) and were ventilated (frequency: 210/min, volume: 250 µl). 30 min before the procedure, buprenorphine (Temgesic, 0.1 mg/kg) was given subcutaneously. Body temperature was assessed using a rectal probe and ECGs were recorded. On day zero a lateral thoracotomy was performed during a preparative surgery. A suture (8-0 monofilament polypropylene suture, Johnson & Johnson, New Jersey, US) was placed around the left anterior descending artery (LAD) and the endings of the suture were tunneled through an Intramedic PE-1 0 tube (Alimed, MA, US). The suture endings were saved under the skin until 7 days after preparative surgery.

### Example 2 Induction of ischemia and reperfusion injury

In order to induce ischemia, the suture was tightened to completely block the blood flow. After 1 hr of ischemia, the suture was loosened and reperfusion was induced. In the experiments, a 1 hr ischemia and a 2 hr reperfusion period was used. Wild-type APC in a dose of 0.4 mg/kg, mutated APC in a dose of 0.4 mg/kg, or placebo (0.9% NaCl) were administered as a repeated intravenous dose, 15 min after ischemia and 5 min after reperfusion. After 2 hours reperfusion time, the animals were anesthetized as described above.

### Example 3: Determination of infarct size

Evans Blue/TTC staining. At the end of the reperfusion period, the LAD was re-occluded and Evans Blue dye (Sigma, US) was injected intravenously and allowed to circulate for 2 minutes. The healthy, perfused tissue stained blue and the area (normally) supplied by the ligated vessel (area at risk, AAR) remained pale. After excision of the heart, it was placed in a brain slicer (Braintree scientific, Inc, US) and frozen at -20°C for 15 minutes. Subsequently the tissue was cut into 0.5 mm thick slices and stained with 2,3,5-triphenyltetrazolium (TTC, Sigma, US) for 20 min at 37°C. Within the AAR, TTC stained the healthy tissue brick red while the area of infarction (AOI) remained pale. Pictures of the slices were taken and were analyzed with Adobe Photoshop CS2 to determine the AOI/AAR ratios.

The results are tabulated below.

**Table I**

| Placebo [% AOI/AAR] | APC wt [% AOI/AAR] | S360A [%AOI/AAR] |
|---|---|---|
| 26.7 | 12.5 | 13.4 |
| 43.5 | 30.7 | 15.7 |
| 38 | 42.5 | 16.6 |
| 36 | 38.5 | 6.8 |
| 35.8 | 39.8 | 14.4 |
| 32.2 | 7 | 9.8 |
| | 20.7 | |
| | 23.6 | |
| | | |
| Mean = 35.37 | Mean = 26.91 | Mean = 12.78 |
| SD = 5.6 | SD = 13.2 | SD = 3.8 |

## Claims

1. Use of an APC with reduced proteolytic activity or a precursor thereof for the preparation of a pharmaceutical composition for the treatment of ischemia reperfusion injury.

2. Use according to claim 1 wherein the APC with reduced proteolytic activity or a precursor thereof is a recombinant APC with reduced proteolytic activity or a precursor thereof.

3. Use according to claims 1 or 2 wherein the APC with reduced proteolytic activity or a precursor thereof is mutated in the active center of the serine protease site of that APC or its precursor.

4. Use according to claims 1 - 3 wherein the APC with reduced proteolytic activity or a precursor thereof is S360A APC or S360A protein C.

5. Activated protein C with a reduced proteolytic activity or a precursor thereof for use in treating ischemia reperfusion injury.

6. Activated protein C or a precursor thereof according to claim 5 wherein the APC with reduced proteolytic activity or a precursor thereof is a recombinant APC with reduced proteolytic activity or a precursor thereof.

7. Activated protein C or a precursor thereof according to claims 5 or 6 wherein the APC with reduced proteolytic activity or a precursor thereof is mutated in the active center of the serine protease site of that APC or its precursor.

8. Activated protein C or its precursor according to claims 5 - 7 wherein the APC with reduced proteolytic activity or a precursor thereof is S360A APC or S360A protein C.

9. Use of an APC with reduced proteolytic activity or a precursor thereof for the preparation of a pharmaceutical composition for the prevention of ischemia reperfusion injury by administering said pharmaceutical composition to a subject at risk for ischemia reperfusion injury.

10. Use according to claim 9 wherein the APC with reduced proteolytic activity or a precursor thereof is a recombinant APC with reduced proteolytic activity or a precursor thereof.

11. Use according to claims 9 or 10 wherein the APC with reduced proteolytic activity or a precursor thereof is mutated in the active center of the serine protease site of that APC or its precursor.

12. Use according to claims 9 - 11 wherein the APC with reduced proteolytic activity or a precursor thereof is S360A APC or S360A protein C.

13. APC with a reduced proteolytic activity or a precursor thereof for preventing ischemia reperfusion injury in a subject at risk for ischemia reperfusion injury.

14. APC or a precursor thereof according to claim 13 which is a recombinant APC with reduced proteolytic activity or a precursor thereof.

15. APC or a precursor thereof according to claims 13 or 14 which is mutated in the active center of the serine protease site of that APC or its precursor.

16. APC or a precursor thereof according to claims 13 - 15 which is S360A APC or S360A protein C.
